# EUROPEAN PATENT SPECIFICATION

(11) **EP 3 411 055 B1**
(45) Date of publication and mention of the grant of the patent: **04.01.2023**
(21) Application number: 17706342.7
(22) Date of filing: 02.02.2017
(51) Int. Cl.: A23K 10/37, A23K 20/20, A23K 20/22, A23K 50/75, A61K 9/46, A61K 36/87, A61K 33/04, A23K 50/30, A23K 50/60

(54) **ANTIOXIDANTS AGAINST OXIDATIVE STRESS DURING A STRESSOR EVENT**
ANTIOXIDANTIA GEGEN OXIDATIVEN STRESS WÄHREND EINER STRESSSITUATION
ANTIOXYDANTS CONTRE UN STRESS OXYDATIF PENDANT UN EVENEMENT STRESSANT

(30) Priority: 05.02.2016 US 201662292192 P
(43) Date of publication of application: 12.12.2018
(73) Proprietor: Nor-Feed, 49070 Beaucouzé (FR)
(72) Inventor: RUTTEN, Alphons, 49240 Avrille (FR); POMMELLET, Caroline, 29660 Carantec (FR); CHICOTEAU, Pierre, 49240 Avrille (FR)
(74) Representative: Bringer IP
(86) International application number: PCT/US2017/016243
(87) International publication number: WO 2017/136570

(56) References cited:
- WO-A1-2012/017363
- US-A1- 2004 115 309
- US-A1- 2013 064 803
- ARCHILE-CONTRERAS A C ET AL: "Oxidative stress may affect meat quality by interfering with collagen turnover by muscle fibroblasts", FOOD RESEARCH INTERNATIONAL, ELSEVIER, AMSTERDAM, NL, vol. 44, no. 2, 1 December 2010 (2010-12-01), pages 582-588, XP028171521, ISSN: 0963-9969, DOI: 10.1016/J.FOODRES.2010.12.002 [retrieved on 2010-12-10]
- Juan Gomez-Basauri: "Impact of Dietary Selenium on Meat Quality", , 1 January 2004 (2004-01-01), XP055365052, Retrieved from the Internet: URL:https://www.google.de/url?sa=t&rct=j&q =&esrc=s&source=web&cd=6&ved=0ahUKEwiw7rOP 0K3TAhXNaVAKHaaGDgoQFghHMAU&url=http://www .meatscience.org/docs/default-source/publi cations-resources/rmc/2004/the-impact-of-d ietary-selenium-on-meat-quality(3).pdf?sfv rsn=2&usg=AFQjCNG8PMHDMhEThoHpATj2-Kj0ecE6 pw [retrieved on 2017-04-18]
- DATABASE GNPD [Online] MINTEL; 9 May 2012 (2012-05-09), anonymous: "Grape Flavoured Energizing Drink Mix", XP055662321, retrieved from www.gnpd.com Database accession no. 1791566

## Description

### FIELD OF THE INVENTION

The present disclosure relates generally to grape extract and selenium antioxidant compositions and methods of using the compositions in animals for improving meat quality.

### BACKGROUND

Impact of Dietary Selenium on Meat Quality » J. Gomez-Basauri (retrieved from the Internet URL:https.//www.google.de/url?sa=t&rct=j&q=&esrc=s&source=web&cd=6&ved=0ahUKEwiw7rOP0K 3TAhXNaVAKHaaGDgoQFghHMAU&url=http://www.meatscience.org/docs/default-source/publications-resources/rmc/2004/the-impact-of-dietary-selenium-on-meat-quality(3).pdf?sfvrsn=2&usg=AFQjCNG8PMHDMhEThoHpATj2-Kj0ecE6pw) provides a summary of knowledges regarding improvement of meat quality, in particular it deals with the role of selenium. According to this document, several studies show that the source of selenium - selenium yeast and sodium selenite are compared- has an impact on the effectiveness on meat production. This document is silent as regards to the use of other ingredients used in the present invention like grape extract, sodium bicarbonate.

### SUMMARY OF THE INVENTION

In a first aspect, the invention is a composition containing sodium bicarbonate, grape extract, an organic acid, and selenium, wherein the composition comprises (a) 10 percent (w/w) to 30 percent (w/w) of grape extract;(b) 30 percent (w/w) to 60 percent (w/w) of sodium bicarbonate;(c) 0.01 percent (w/w) to 0.50 percent (w/w) of selenium; (d) 0.25 percent (w/w) to 2.0 percent (w/w) of a lubricant; (e) 20 percent (w/w) to 40 percent (w/w) dry organic acid ;and (f) optionally, 1.0 percent (w/w) to 3.0 percent (w/w) of a binder.

In one embodiment, the selenium is derived from an organic source, such as yeast. In another embodiment, the selenium is derived from an inorganic source, such as sodium selenite.

In a second aspect, the invention is a method for producing meat comprising raising an animal on a diet that is supplemented with a composition according to the first aspect.

### BRIEF DESCRIPTION OF THE DRAWINGS

### DETAILED DESCRIPTION OF THE INVENTION

In one aspect, the invention is a composition containing sodium bicarbonate, organic acid, grape extract, and selenium, wherein the grape extract is 10 percent (w/w) to 30 percent (w/w) of the composition; sodium bicarbonate is 30 percent (w/w) to 60 percent (w/w) of the composition; the organic acid is dry organic acid which is 20 percent (w/w) to 40 percent (w/w) of the composition; and the selenium is 0.01 percent (w/w) to 0.5 percent (w/w) of the composition. The composition also includes 0.25 percent (w/w) to 2.0 percent (w/w) of a lubricant. In some embodiments, the amount of lubricant is 0.5 percent (w/w) to 1.0% (w/w). Lubricants are ingredients added in small amounts to improve processing such as to prevent ingredients from clumping together and to prevent sticking to a tablet making apparatus. Lubricants known in the art of tablet manufacture, such as glyceryl tristearate, stearic acid, and talc may be included in certain embodiments of the invention. In some embodiments of the invention, the lubricant is magnesium stearate.

The instant invention is an effervescent composition, which in one preferred embodiment is in the form of a tablet, alternatively called a disc or puck. The effervescent quality is provided by the presence of sodium bicarbonate and organic acid, which produces carbon dioxide bubbles when the composition is added to water. As would be appreciated by one skilled in the art, sodium bicarbonate and organic acid will have an effect on the pH of the solution that is formed when the tablet is dissolved in water. In addition, the effervescence will aid the dissolution of the tablet due to the turbulence of gas bubble formation. In certain powder or tablet embodiments of the instant invention, the organic acid is dry organic acid. In some embodiments, the organic acid comprises at least one acid selected from group consisting of citric acid, fumaric acid, or tartaric acid. In one embodiment the organic acid preferably comprises citric acid.

The composition optionally contains 1.0 percent (w/w) to 3.0 percent (w/w) of a binder. By binder is meant a substance which helps to hold a tablet together. Non-limiting representative binders include acacia, cellulose, gelatin, methyl cellulose, starch, polyvinylpyrrolidone, and polyethylene glycol. Other pharmaceutically acceptable excipients known to those skilled in the art to be suitable for manufacture of tablets are optionally included in some embodiments of the invention. These excipients may be, for example, inert diluents, such as calcium carbonate, sodium carbonate, lactose, calcium phosphate or sodium phosphate; granulating and disintegrating agents, for example, corn starch, or alginic acid. Other additives such as preservatives, colorants, and solubility enhancers, may optionally be included in certain embodiments of the instant invention.

In one embodiment, the selenium is organically derived. In another embodiment, the selenium is inorganic selenium. In yet another embodiment, selenium is a combination of inorganic and organically derived selenium. Organic selenium may be in the form of selenomethione (SeMet). In certain embodiments, the organically derived selenium is from yeast such as a strain of the yeast *Saccharomyces cerevisiae* grown in a sodium selenite medium. In another embodiment, the selenium is sodium selenite. In another embodiment the inorganic selenium is calcium selenite. In some embodiments, the selenium concentration in the composition is 0.05 % to 0.20 % (w/w). In another embodiment, the concentration of selenium is 0.01 to 0.10% (w/w). In one embodiment, the composition preferably contains 506 mg/kg selenium; in another embodiment, the composition preferably contains 0.11% (w/w) sodium selenite.

The minimum nutrient requirement for selenium in animals is typically in the range of 0.1-0.3 mg/kg. Selenosis, toxicity due to selenium, is known to occur when selenium is consumed at significantly higher amounts than the minimum nutrient requirement. The levels of selenium provided by the instant invention are well below levels considered to be toxic. Table 1 shows representative typical daily water consumption levels for several species of animals, and the amount of selenium that would be provided by one exemplary embodiment of the instant invention.

**Table 1: 10g Effervescent Grape Extract Tablet (20% Grape Extract, 506 mg/kg selenium, 48.41% sodium bicarbonate, 0.5% magnesium stearate) mixed with 100 liters of drinking water**

| Species | Selenium (in mg) | Daily Water Consumption (in liters) |
|---|---|---|
| Rabbit | 0.05 | 0.5 |
| Chicken | 0.005-0.006 | 0.09-0.12 |
| Piglet | 0.15 | 3 |

In some embodiments, the grape extract in the instant invention is a powder obtained by extraction of grape (*Vitis vinifera*) with water and alcohol. After extraction the grape extract is dried to provide a powder, using a technique such as spray drying. Other methods of drying an extract that are known in the art, including freeze-drying, are suitable for some embodiments. In certain embodiments, the grape extract is preferably rich in polyphenols (>80%) and procyanidols (> 60%), and contains anthocyanins (> .75%).

In one aspect, the instant invention provides a method of producing meat with improved quality comprising raising an animal on a diet that is supplemented with a composition comprising:
(a) 10 percent (w/w) to 30 percent (w/w) of grape extract;
(b) 30 percent (w/w) to 60 percent (w/w) of sodium bicarbonate;
(c) 0.01 percent (w/w) to 0.50 percent (w/w) of selenium;
(d) 0.25 percent (w/w) to 2.0 percent (w/w) of a lubricant;
(e) and 20 percent (w/w) to 40 percent (w/w) of dry organic acid; and
(f) optionally, 1.0 percent (w/w) to 3.0 percent (w/w) of a binder.

In some embodiments of the invention, the composition of sodium bicarbonate is 40 to 60 percent (w/w). In some embodiments, the concentration of lubricant is 0.50 to about 1.0 percent (w/w). In some embodiments, the dry organic acid comprises at least one acid selected from the group consisting of citric acid, fumaric acid, and tartaric acid. In one embodiment, the dry organic acid preferably comprises citric acid.

In one embodiment, the concentration of selenium is about 0.05 percent (w/w) to 0.2 percent (w/w). In one embodiment, the concentration of selenium is 0.01 percent (w/w) to 0.1 percent (w/w). In one embodiment, the composition preferably contains 506 mg/kg selenium; in another embodiment, the composition preferably contains 0.11% (w/w) sodium selenite.

In one embodiment of the invention, the composition is administered in drinking water. In another embodiment, the concentration of the composition in the drinking water is preferably 0.05 % (w/v) to 0.2% (w/v). As would be appreciated by one having general knowledge in the art, the composition may be simply added to the drinking water in one step. Alternatively, it may be preferred, in some cases, to add the composition to a first quantity of water until it is dissolved to form a concentrate, then the concentrate may be diluted by adding it to a reservoir, or second quantity, of water so as to provide drinking water containing the composition at the desired concentration.

Meat producers are motivated to provide a high quality product that meets the demands of the consumer, particularly when they can obtain a higher price for the meat based on a particular quality criterion. For example, the quality of poultry meat is of particular importance to consumers and poultry producers alike, and the qualitative value of the meat is commonly assessed by measuring water loss, pH, meat color, and other sensory characteristics.

In one embodiment of the invention, the quality of the meat is measured by determining the water loss rate, which is decreased in meat from an animal raised on a diet supplemented with the composition as compared to meat from an animal that is raised on a diet that is not supplemented with the composition.

Compositions of the instant invention surprisingly have a significant effect on the water holding capacity of poultry broiler meat. Due to the improved water holding capacity, the water loss rate, also called the exudation rate, is decreased. The rate of water loss may be determined under standard conditions, such as by determining the percentage of moisture lost over time during storage under refrigeration, or during cooking for a period of time. Less moisture loss during cooking is advantageous because it provides a juicer cooked product that is desirable to consumers.

Without being bound by theory, it is believed that the effervescent grape extract/Se compositions according to the invention contribute to better antioxidant status and overall better animal health, resulting in optimized development of the flesh and improved water holding capacity.

### EXAMPLES

Below, the presently disclosed invention will be described by way of examples, which are provided for illustrative purposes only and accordingly are not to be construed as limiting the invention.

### Example 1

### Efficacy in vaccinated chickens

The effectiveness of the invention was tested in broiler chicks (*Gallus gallus*). The composition of the invention was administered six days prior to a stressor event (*i.e.,* vaccination) for four consecutive days and compared with a control. The effect on the consumption of water and food, growth (live weight), feed efficiency, antioxidant status, quality of the meat and mortality was measured.

### General Procedure

### Acclimation period

All animals (1,680 broilers + spare animals) were housed at a test facility (CEBIPHAR, Fondettes, France; www.cebiphar.com) and examined for any sign of disease or abnormality. The animals were observed daily for signs of any disease throughout the acclimation period. They were individually identified with a unique number using wing clips, weighed and allocated to pens and groups according to Table 1. There were 42 broilers in each cage and each cage was assigned a treatment as explained below.

**Table 2.**

| | | | |
|---|---|---|---|
| **No. of cage** | **Treatment** | **Sex** | **Area** |
| **1** | T4 | M | 1 |
| **2** | T3 | M | 1 |
| **3** | T2 | M | 1 |
| **4** | T1 | M | 1 |
| **5** | T4 | F | 1 |
| **6** | T3 | F | 1 |
| **7** | T2 | F | 1 |
| **8** | T1 | F | 1 |
| **9** | T4 | M | 1 |
| **10** | T3 | M | 1 |
| **11** | T1 | M | 1 |
| **12** | T2 | M | 1 |
| **13** | T3 | F | 1 |
| **14** | T1 | F | 1 |
| **15** | T2 | F | 1 |
| **16** | T4 | F | 1 |
| **17** | T4 | M | 2 |
| **18** | T1 | M | 2 |
| **19** | T2 | M | 2 |
| **20** | T3 | M | 2 |
| **21** | T4 | F | 2 |
| **22** | T1 | F | 2 |
| **23** | T2 | F | 2 |
| **24** | T3 | F | 2 |
| **25** | T4 | M | 2 |
| **26** | T2 | M | 2 |
| **27** | T1 | M | 2 |
| **28** | T3 | M | 2 |
| **29** | T4 | F | 2 |
| **30** | T2 | F | 2 |
| **31** | T1 | F | 2 |
| **32** | T3 | F | 2 |
| **33** | T3 | M | 3 |
| **34** | T1 | M | 3 |
| **35** | T2 | M | 3 |
| **36** | T4 | M | 3 |
| **37** | T3 | F | 3 |
| **38** | T2 | F | 3 |
| **39** | T1 | F | 3 |
| **40** | T4 | F | 3 |

Spare animals were housed in the same building.

### Treatment

The treatment was a composition in a powder form for veterinary use as a supplement added to drinking water. The treatment composition is described in Table 3.

**Table 3.**

| Substance | %w/w | Quantity in grams (per 10 grams) |
|---|---|---|
| Sodium selenite (46% selenium)¹ | 0.11% | 0.011g |
| Grape extract | 20% | 2.000g |
| Sodium bicarbonate | 48.20% | 4.820g |
| Citric acid | 30.85% | 3.085g |
| Magnesium stearate | 0.84% | 0.084g |
| Total | 100.00% | 10.000g |

| | | |
|---|---|---|
| Note 1. 46% is the percentage that is pure selenium | | |

The treatment composition was administered daily in the drinking water from D6 (day 6) to D10 (day 10) at a dose as described in Table 4.

**Table 4.**

| Lot Description | Dosage |
|---|---|
| Lot 1 (T1) | 10 grams / 100 liters water |
| Lot 2 (T2) | 10 grams / 50 liters water |
| Lot 3 (T3) | 10 grams / 200 liters water |
| Lot 4 (T4) | 0 grams (control) |

The supplemented water in each drinker was refreshed on each treatment day and fresh solution containing the treatment composition was prepared each day of treatment before administration to the animals. Treatment was given approximately at the same time in the morning each day throughout the study.

### Application of a Stressor (Vaccination)

Two stressors in the form of vaccines were used. The first was Gallivac IB88 vaccine for infectious bronchitis administered via nebulization to the 1,680 test subject broilers on D13. The second was HipraGumboro G97 vaccine administered via drinking water to the 1,680 test subject broilers at D17. Both vaccines were administered according to manufacturer's instructions.

### Clinical observations

The animals were clinically observed daily for signs of illness throughout the study.

### Water intake

Water consumption of each of the subgroups was recorded every day for two weeks to include the week prior to the vaccination and the week following vaccination.

### Feed intake

Feed was weighed on a calibrated scale and values recorded on a specific data capture form. Cumulative consumption of food was recorded at D6, D17 and D35.

### Body weight

Body weight (collective as per cage) was recorded on D0, D6, D17, and D35.

### Blood collection

Six percent of the tested animals were sampled for blood from a wing vein. The first sample was collected at arrival to obtain a baseline reading of the oxidative stress indicators (see below). Second and third samples were taken on D20 and D35.

Blood was collected into 2 mL tubes from the wing vena of the animals in compliance with CEBIPHAR standard operating procedures. For each sampled animal, 1 EDTA tube and 1 heparinated tube filled up to 1 ml each was used (*i*.*e*.. 2 ml collected in total per animal). Blood was collected by blood-letting (sacrifice of the chicken) if needed. All tubes were labeled with the CEBIPHAR study number, the animal number, the group, the day of collection and the content. Caution was taken to reduce light exposure of the samples. The tubes were not processed prior to being tested (i.e., no centrifugation, no pooling of samples). No treatments other than indicated in the study plan were administered during the course of the study. Blood samples were shipped to a testing organization within 24 hours in a refrigerated container for analysis.

### End of the Study

Field tests ended at the last weighing and bloodletting of the broilers before slaughter. Fifty two broilers were slaughtered in a slaughterhouse approved for analysis of breast filets and legs in the laboratory, and analysis was conducted on the exudate (water loss), pH and color.

The exudate was measured as follows. The pectoralis major (PM) muscle is weighed (Pi = initial weight), placed in a plastic bag zipper and suspended by a hook (planted in the upper part of the muscle) for seven days at + 2 to + 4 ° C. The PM muscles was then wiped dry with paper and weighed again (Pf = final weight after exudation) in order to assess the amount of water lost during the storage of the product. The losses are expressed in percentage of the initial weight.

The pH of breasts filets was analyzed the day after slaughter. The color analysis of the breast filets was conducted seven days after slaughter.

### Results

Forty pens were prepared to accommodate 42 chicks each (minimum surface area 2.37 square meters). The chicks were sexed upon arrival and distributed to the pens. Each pen contained one round trough and one hanging feeder.

The forty pens were divided into four treatment groups as outlined above. The number of males and females for each treatment group was equalized.

### Effect on Growth (Live Weight)

There is a significant treatment effect for male birds only as measured at D6 and D35 of age shown in Table 5.

**Table 5.**

| Treatment | D6 Weight (grams) | D35 Weight (grams) |
|---|---|---|
| 1 (10g/100L) | 162 | 2376 |
| 2 (10g/50L) | 165 | 2366 |
| 3 (10g/200L) | 168 | 2419 |
| 4 (control) | 168 | 2444 |

| | | |
|---|---|---|
| ^{∗}p=0.05 at D6 and p=0.001 at D35. | | |

### Effect on Meat Quality

There is also a significant treatment effect in the meat quality as measured by exudation of the breast filets shown in Table 6.

**Table 6.**

| Treatment | Exudation of breast filets |
|---|---|
| 1 (10g/100L) | 3.24% |
| 2 (10g/50L) | 3.86% |
| 3 (10g/200L) | 4.74% |
| 4 (control) | 4.08% |

| | |
|---|---|
| ^{∗}p=0.008 | |

### Antioxidant Status for illustration purposes only

Several antioxidant related parameters were measured to include hydrophilic total antioxidant capacity (CATH), lipophilic total antioxidant capacity (CATL), glutathione peroxidase on whole blood (GPX), superoxide dismutase (SOD), peroxides plasma lipids (POOL), protein plasma lipids (PROTOX) and vitamin E. The antioxidant related parameters in blood samples were measured using a battery of oxidative stress assays (Analyses De Stress Oxydant (Oxidative Stress Analysis) Laboratory Frank Duncombe. http://www.labo-frank-duncombe.fr/). At 20 days of age, a significant dosage effect was measured for CATH at the grouped high dosage administration (*i.e.,* Lot 1 + Lot 2 = 946.9 µmol) compared with the grouped low dosage administration (*i.e.,* Lot 3 + Lot 4 = 843.4 µmοl; ^{∗}p=0.005). No other parameters measured showed a significant treatment effect.

Other parameters measured but displaying no significant treatment effect included food consumption, water consumption, mortality, pH and color of the breast filets.

## Claims

1. A composition comprising:
(a) 10 percent (w/w) to 30 percent (w/w) of grape extract;
(b) 30 percent (w/w) to 60 percent (w/w) of sodium bicarbonate;
(c) 0.01 percent (w/w) to 0.50 percent (w/w) of selenium;
(d) 0.25 percent (w/w) to 2.0 percent (w/w) of a lubricant;
(e) 20 percent (w/w) to 40 percent (w/w) dry organic acid; and
(f) optionally, 1.0 percent (w/w) to 3.0 percent (w/w) of a binder.

2. The composition of claim 1 wherein the selenium is organically derived or is a sodium selenite salt.

3. The composition of claim 1 or 2 wherein the selenium is present in an amount of 0.01 percent (w/w) to t 0.2 percent (w/w).

4. A method of producing meat with improved quality comprising raising an animal on a diet that is supplemented with a composition according to any of claims 1-3.

5. A method of producing meat with improved quality according to claim 4, wherein said composition is administered in drinking water, preferably wherein the concentration of the composition in drinking water is 0.05 percent (w/v) to 0.2 percent (w/v).

6. A method of producing meat with improved quality according to claim 4 or 5, wherein the quality of the meat is measured by determining the water loss rate, which is decreased in meat from an animal raised on a diet supplemented with the composition as compared to meat from an animal that is raised on a diet that is not supplemented with the composition.

## Patentansprüche

1. Zusammensetzung, welche umfasst:
(a) 10 bis 30 Prozent (w/w) Traubenextrakt;
(b) 30 bis 60 Prozent (w/w) Natriumbicarbonat;
(c) 0,01 bis 0,50 Prozent (w/w) Selen;
(d) 0,25 bis 2,0 Prozent (w/w) eines Schmiermittels;
(e) 20 bis 40 Prozent (w/w) trockene organische Säure; und
(f) wahlweise 1,0 bis 3,0 Prozent (w/w) eines Bindemittels.

2. Zusammensetzung nach Anspruch 1, worin das Selen organisch gewonnen ist oder ein Natriumselenitsalz ist.

3. Zusammensetzung nach Anspruch 1 oder 2, worin das Selen in einer Menge von 0,01 bis 0,2 Prozent (w/w) vorliegt.

4. Verfahren zur Erzeugung von Fleisch mit verbesserter Qualität, wobei ein Tier mit einem Futter aufgezogen wird, das mit einer Zusammensetzung nach einem der Ansprüche 1 bis 3 ergänzt wurde.

5. Verfahren zur Erzeugung von Fleisch mit verbesserter Qualität nach Anspruch 4, wobei die Zusammensetzung im Trinkwasser verabreicht wird, vorzugsweise wobei die Konzentration der Zusammensetzung im Trinkwasser 0,05 Prozent (w/v) bis 0,2 Prozent (w/v) beträgt.

6. Verfahren zur Herstellung von Fleisch mit verbesserter Qualität nach Anspruch 4 oder 5, wobei die Qualität des Fleisches durch Bestimmung der Wasserverlustrate gemessen wird, die bei Fleisch von einem Tier, das mit einer mit der Zusammensetzung ergänzten Diät aufgezogen wurde, verringert ist, im Vergleich zu Fleisch von einem Tier, das mit einer nicht mit der Zusammensetzung ergänzten Diät aufgezogen wurde.

## Revendications

1. Composition comprenant :
(a) 10 pour cent (p/p) à 30 pour cent (p/p) d'extrait de raisin ;
(b) 30 pour cent (p/p) à 60 pour cent (p/p) de bicarbonate de sodium ;
(c) 0,01 pour cent (p/p) à 0,50 pour cent (p/p) de sélénium ;
(d) 0,25 pour cent (p/p) à 2,0 pour cent (p/p) d'un lubrifiant ;
(e) 20 pour cent (p/p) à 40 pour cent (p/p) d'acide organique sec ; et
(f) facultativement, 1,0 pour cent (p/p) à 3,0 pour cent (p/p) d'un liant.

2. Composition selon la revendication 1, dans laquelle le sélénium est dérivé organiquement ou est un sel de sélénite de sodium.

3. Composition selon la revendication 1 ou 2, dans laquelle le sélénium est présent en une quantité de 0,01 pour cent (p/p) à 0,2 pour cent (p/p).

4. Procédé de production de viande de qualité améliorée comprenant le fait d'élever un animal avec un régime qui est supplémenté avec une composition selon l'une quelconque des revendications 1 à 3.

5. Procédé de production de viande de qualité améliorée selon la revendication 4, dans lequel ladite composition est administrée dans l'eau potable, de préférence dans lequel la concentration de la composition dans l'eau potable est de 0,05 pour cent (p/v) à 0,2 pour cent (p/v).

6. Procédé de production de viande de qualité améliorée selon la revendication 4 ou 5, dans lequel la qualité de la viande est mesurée en déterminant le taux de perte d'eau, qui est diminué dans la viande d'un animal élevé avec un régime supplémenté avec la composition par comparaison avec la viande d'un animal qui est élevé avec un régime qui n'est pas supplémenté avec la composition.
